# EUROPEAN PATENT APPLICATION

(11) **EP 4 609 838 A1**
(43) Date of publication of application: **03.09.2025**
(21) Application number: 24815808.1
(22) Date of filing: 24.05.2024
(51) Int. Cl.: A61F 9/00, A61B 3/00, A61B 3/02

(54) **APPARATUS AND METHOD FOR PROVIDING VISUAL PERCEPTION TRAINING USING CIRCULAR LAYER COORDINATE SYSTEM**

(30) Priority: 26.05.2023 KR 20230068464
(71) Applicant: NUNAPS INC., Seoul 05505 (KR)
(72) Inventor: KANG, Dong Wha, Seoul 05505 (KR); CHOI, Hagyun, Seoul 05505 (KR)
(74) Representative: dompatent von Kreisler Selting Werner - Partnerschaft von Patent- und Rechtsanwälten mbB
(86) International application number: PCT/KR2024/007114
(87) International publication number: WO 2024/248432

(57) **Abstract**

Disclosed are a visual perception training providing apparatus and a method thereof. The visual perception training providing apparatus includes a display module that displays a circular layer coordinate system, which includes a plurality of circular layers and a plurality of straight lines, and which includes a plurality of cross points where the plurality of circular layers intersect the plurality of straight lines, a memory that stores information about the circular layer coordinate system, an input module that receives a response of a trainee, and a control module that calculates response scores of the trainee for the plurality of cross points in order from a circular layer closest to an origin of the circular layer coordinate system to a circular layer furthest away from the origin, sets a predetermined number of a training point based on the calculated response scores, and performs visual perception training on the training point.

## Description

### [TECHNICAL FIELD]

Embodiments of the present disclosure described herein relate to a visual perception training providing apparatus and a method thereof, and more particularly, relate to an apparatus for providing visual perception training by using a circular layer coordinate system, and a method thereof.

### [BACKGROUND ART]

Many patients experience discomfort due to a visual impairment or impaired visual ability caused by eye diseases such as glaucoma, macular degeneration, and diabetic retinopathy. The eye diseases may cause abnormalities in a visual system, such as a problem in a function of an optic nerve due to pressure on the optic nerve or failure of blood supply.

In the past, drugs have been injected or surgery has been performed to treat these diseases, but treatment may not proceed smoothly.

Accordingly, there is continuously required for an effective method for improving a visual ability. A device and method for improving such the visual ability by performing visual perception learning has emerged.

### [DETAILED DESCRIPTION OF THE INVENTION]

### [TECHINICAL PROBLEM]

Embodiments of the present disclosure provide an apparatus for providing visual perception training by using a circular layer coordinate system, and a method thereof.

Problems to be solved by the present disclosure are not limited to the problems mentioned above, and other problems not mentioned will be clearly understood by those skilled in the art from the following description.

### [TECHNICAL SOLUTION]

According to an embodiment, a visual perception training providing apparatus includes a display module that displays a circular layer coordinate system, which includes a plurality of circular layers and a plurality of straight lines, and which includes a plurality of cross points where the plurality of circular layers intersect the plurality of straight lines, a memory that stores information about the circular layer coordinate system, an input module that receives a response of a trainee, and a control module that calculates response scores of the trainee for the plurality of cross points in order from a circular layer closest to an origin of the circular layer coordinate system to a circular layer furthest away from the origin, sets a predetermined number of a training point based on the calculated response scores, and performs visual perception training on the training point.

According to an embodiment, a visual perception training providing method includes displaying a circular layer coordinate system, which includes a plurality of circular layers and a plurality of straight lines, and which includes a plurality of cross points where the plurality of circular layers intersect the plurality of straight lines, on a display module, calculating response scores of a trainee for the plurality of cross points in order from a circular layer closest to an origin of the circular layer coordinate system to a circular layer furthest away from the origin, setting a predetermined number of a training point based on the calculated response scores, and performing visual perception training on the training point.

### [ADVANTAGEOUS EFFECTS OF THE INVENTION]

In accordance with the visual perception training providing apparatus 100 according to an embodiment of the present disclosure, it is possible to set a training point, at which visual perception is capable of being achieved at a specific level, and to provide visual perception training on the corresponding training point, thereby effectively increasing the trainee's visual ability.

Moreover, in accordance with the visual perception training providing apparatus 100 according to an embodiment of the present disclosure, it is possible to expand the trainee's visual perception range by resetting the training point depending on the training result.

### [DESCRIPTION OF THE DRAWINGS]

FIG. 1 is a block diagram of a visual perception training providing apparatus, according to an embodiment of the present disclosure;
FIG. 2 is a diagram explaining a circular layer coordinate system, according to an embodiment of the present disclosure;
FIG. 3 is a diagram for describing a training point setting operation using a circular layer coordinate system, according to an embodiment of the present disclosure;
FIG. 4 is a diagram illustrating a training point setting operation in a first circular layer, according to an embodiment of the present disclosure;
FIG. 5 is a diagram illustrating a training point setting operation in a second circular layer, according to an embodiment of the present disclosure;
FIG. 6 is a diagram illustrating a training point setting operation when two or more candidates are found, according to an embodiment of the present disclosure;
FIG. 7 is a diagram for describing a training point setting operation when one candidate is found, according to an embodiment of the present disclosure;
FIGS. 8 to 10 are diagrams for describing a training point setting operation when there is no candidate, according to an embodiment of the present disclosure;
FIG. 11 is a diagram showing a size of a reference image for each circular layer, according to an embodiment of the present disclosure; and
FIG. 12 is a flowchart showing a visual perception training providing method, according to an embodiment of the present disclosure.

### [BEST MODE]

The same reference numerals denote the same elements throughout the present disclosure. The present disclosure does not describe all elements of embodiments. Well-known content or redundant content in which embodiments are the same as one another will be omitted in a technical field to which the present disclosure belongs. A term such as 'unit, module, member, or block' used in the specification may be implemented with software or hardware. According to embodiments, a plurality of 'units, modules, members, or blocks' may be implemented with one component, or a single 'unit, module, member, or block' may include a plurality of components.

Throughout this specification, when it is supposed that a portion is "connected" to another portion, this includes not only a direct connection, but also an indirect connection. The indirect connection includes being connected through a wireless communication network.

Furthermore, when a portion "comprises" a component, it will be understood that it may further include another component, without excluding other components unless specifically stated otherwise.

Throughout this specification, when it is supposed that a member is located on another member "on", this includes not only the case where one member is in contact with another member but also the case where another member is present between two other members.

Terms such as 'first', 'second', and the like are used to distinguish one component from another component, and thus the component is not limited by the terms described above.

Unless there are obvious exceptions in the context, a singular form includes a plural form.

In each step, an identification code is used for convenience of description. The identification code does not describe the order of each step. Unless the context clearly states a specific order, each step may be performed differently from the specified order.

Hereinafter, operating principles and embodiments of the present disclosure will be described with reference to the accompanying drawings.

In this specification, an 'apparatus according to an embodiment of the present disclosure' includes all various devices capable of providing results to a user by performing arithmetic processing. For example, the apparatus according to an embodiment of the present disclosure may include all of a computer, a server device, and a portable terminal, or may be in any one form.

Here, for example, the computer may include a notebook computer, a desktop computer, a laptop computer, a tablet PC, a slate PC, and the like, which are equipped with a web browser.

The server device may be a server that processes information by communicating with an external device and may include an application server, a computing server, a database server, a file server, a game server, a mail server, a proxy server, and a web server.

For example, the portable terminal may be a wireless communication device that guarantees portability and mobility, and may include all kinds of handheld-based wireless communication devices such as a smartphone, a personal communication system (PCS), a global system for mobile communication (GSM), a personal digital cellular (PDC), a personal handyphone system (PHS), a personal digital assistant (PDA), International Mobile Telecommunication (IMT)-2000, a code division multiple access (CDMA)-2000, W-Code Division Multiple Access (W-CDMA), and Wireless Broadband Internet terminal (Wibro) terminal, and a wearable device such as a timepiece, a ring, a bracelet, an anklet, a necklace, glasses, a contact lens, or a head-mounted device (HMD).

Functions related to artificial intelligence according to an embodiment of the present disclosure are operated through a processor and a memory. The processor may consist of one or more processors. In this case, the one or more processors may be a general-purpose processor (e.g., a CPU, an AP, or a digital signal processor (DSP)), a graphics-dedicated processor (e.g., a GPU or a vision processing unit (VPU)), or an artificial intelligence (AI)-dedicated processor (e.g., an NPU). Under control of the one or more processors, input data may be processed depending on an AI model, or a predefined operating rule stored in the memory. Alternatively, when the one or more processors are AI-dedicated processors, the AI-dedicated processor may be designed with a hardware structure specialized for processing a specific AI model.

In this specification, perceptual learning refers to learning for improving the perception of a stimulus through repetitive training on the stimulus. In other words, in this specification, visual perception learning refers to learning for improving the perception of a visual stimulus through repetitive training with a visual stimulus. As a result of the visual perception learning, learners may see things they couldn't see before or may notice differences they couldn't distinguish before. In addition to learning for improving the ability to find objects from a stimulus that comes from the outside through a visual organ, the visual perception learning should be broadly interpreted to include learning for improving the ability to discriminate objects from the stimulus.

In this specification, a visual field defect/visual field disorder refers to the disorder of a visual system from a retina to a cerebral cortex or the abnormality of a visual field. For example, the visual field defect should be broadly interpreted to include not only a visual field defect due to a brain function damage but also a visual field defect due to the death (or malfunction) of a retinal ganglion cell. Non-limiting examples of diseases that cause the visual field disorder may include glaucoma, macular degeneration, diabetic retinopathy, hemianopia, and quadrantanopia.

In this specification, in addition to vision measured based on whether letters, numbers, or symbols on an eyesight test chart may be identified in order of size, the visual ability should be broadly interpreted as a concept that includes a visual perception ability related to object recognition in a brain. For example, eyes normally accept visual stimuli. However, an object is not perceived because the brain fails to perceive the visual stimulus. This may mean that the visual ability is degraded or bad.

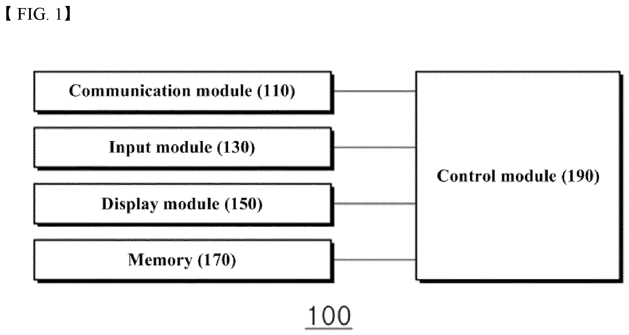

FIG. 1 is a block diagram of a visual perception training providing apparatus, according to an embodiment of the present disclosure. Referring to FIG. 1, a visual perception training providing apparatus 100 may include a communication module 110, an input module 130, a display module 150, a memory 170, and a control module 190. The components shown in FIG. 1 are not essential in implementing the visual perception training providing apparatus 100. The visual perception training providing apparatus 100 described herein may have more or fewer components than those listed above.

The visual perception training providing apparatus 100 according to an embodiment of the present disclosure may include various devices capable of performing calculation processing. For example, the visual perception training providing apparatus 100 may include a desktop PC, a mobile phone, a smart phone, a laptop computer, personal digital assistants (PDA), a portable multimedia player (PMP), a slate PC, a tablet PC, an ultrabook, a wearable device, and the like.

For example, the visual perception training providing apparatus 100 may include a head-mounted device such as a head mounted display (HMD) mounted on a trainee's head to display an image, smart glasses, or smart goggles, or a display device of a mobile phone capable of being mounted and used on the head-mounted device.

The communication module 110 performs wired or wireless communication with at least one external device (a server, etc.). In particular, when wireless communication is performed, wireless signals are exchanged over a communication network based on wireless Internet technologies.

For example, the wireless Internet technologies includes wireless LAN (WLAN), Wireless-Fidelity (Wi-Fi), Wi-Fi Direct, digital living network alliance (DLNA), wireless broadband (WiBro), world interoperability for microwave access (WiMAX), high speed downlink packet access (HSDPA), high speed uplink packet access (HSUPA), long term evolution (LTE), long term evolution-advanced (LTE-A), and the like. The visual perception training providing apparatus 100 transmits and receives data depending on at least one wireless Internet technology within a range including Internet technologies not listed above.

The communication module 110 may be used for short range communication, and may support short-range communication by using at least one of Bluetooth^{™}, radio frequency identification (RFID), infrared data association (IrDA), ultra wideband (UWB), ZigBee, near field communication (NFC), Wireless-Fidelity (Wi-Fi), Wi-Fi Direct, and wireless universal serial bus (Wireless USB) technologies. In this case, the short range wireless communication network may be wireless personal area networks.

The input module 130 may obtain a signal corresponding to a trainee's input. For example, the input module 130 may obtain the trainee's input for performing a training point setting operation or visual perception training, a response to a training point setting operation and a visual perception training operation, which are provided through the display module 150, or the like.

In this case, the input module 130 may include a keyboard, a keypad, a button, a jog shuttle, a wheel, and the like. Besides, the trainee's input in the input module 130 may be, for example, pressing a button, touching, and dragging.

The input module 130 may be configured as a separate module connected to the visual perception training providing apparatus 100 in a wired or wireless manner. For example, the visual perception training providing apparatus 100 may provide a trainee with images for setting a training point or performing visual perception training through the display module 150 mounted on the trainee's head, and may receive a response from the trainee through the input module 130 implemented as a separate module given to the trainee's hand.

The display module 150 outputs videos or images. For example, the display module 150 may include an LCD, an OLED display, an AMOLED display, and the like. In an embodiment, when the display module 150 is implemented as a touch screen, the display module 150 may serve as the input module 130. In this case, the separate input module 130 may not be provided depending on a selection, or the input module 130 performing limited functions such as a volume control button, a power button, and a home button may be provided. Moreover, the display module 150 may be provided in a form of a video output port that transmits image information to an external display device.

In an embodiment, the display module 150 may include a first display and a second display, which respectively correspond to the trainee's eyes (a left eye and a right eye). Here, the first display may output a first image, and the second display may output a second image.

In the meantime, the present disclosure is not limited thereto, and the display module 150 may be implemented as a single display. In this case, the display module 150 may output the first image in a left area of the display and may output the second image in a right area of the display.

The memory 170 stores various pieces of data (information) as well as at least a piece of data (information) and at least one process, which are necessary to provide a visual perception training method. For example, a program for performing training, at least a piece of trainee information (personal information, visual information, response information, training results, or the like), a measurement image, a training image, or the like may be stored as data. Besides, the memory 170 may store various instructions, algorithms, or the like to perform the training point setting operation and the visual perception training operation.

Moreover, the memory 170 may include a storage medium of at least one type of a flash memory type, a hard disk type, a multimedia card micro type, a card type memory (e.g., SD memory, XD memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, an optical disc, etc. Besides, the memory 170 may store information temporarily, permanently, or semi-permanently, and may be provided in an embedded type or a removable type.

The control module 190 may control configurations within the visual perception training providing apparatus 100 or may process and calculate various pieces of information. Under control of the control module 190, the training point setting operation and the visual perception training operation may be performed based on at least one process stored in the memory 170.

Before performing the visual perception training operation, the control module 190 may perform a training point setting operation for visual perception training of a trainee. In an embodiment, the control module 190 may set the trainee's training point by using a circular layer coordinate system. Here, the circular layer coordinate system refers to a coordinate system composed of a plurality of circular layers and a plurality of straight lines with different radius sizes based on the origin.

In detail, the control module 190 may display the circular layer coordinate system through the display module 150 and may determine whether the trainee is capable of visually perceiving a plurality of points in the circular layer coordinate system. Also, the control module 190 may select a point, which belongs to a type of gray zone capable of being visually perceived at a specific level, as a training point.

Besides, the control module 190 may perform the trainee's visual perception training operation on the selected training point. In particular, the control module 190 may perform a visual perception training operation of increasing the trainee's visual ability on the selected training point. In addition, the control module 190 may perform a resetting operation on the training point based on the training result of the visual perception training operation.

For example, when the training result for the existing training point is excellent, the control module 190 may set a new training point to further expand the trainee's visual perception range. Alternatively, when the training result for the existing training point is insufficient, the control module 190 may set another training point capable of expanding the visual perception range.

The control module 190 may be implemented with software, hardware, or a combination thereof. For example, in a hardware manner, the control module 190 may be implemented with a field programmable gate array (FPGA) or an application specific integrated circuit (ASIC), a semiconductor chip, or other various types of electronic circuits. Also, for example, in a software manner, the control module 190 may be implemented with various computer languages or a logic program executed depending on the above-described hardware.

Unless otherwise stated in the following description, it may be understood that an operation of the visual perception training providing apparatus 100 is performed under the control of the control module 190.

As such, the visual perception training providing apparatus 100 according to an embodiment of the present disclosure may set a training point, at which visual perception is capable of being achieved at a specific level, and may provide visual perception training on the corresponding training point, thereby effectively increasing the trainee's visual ability.

Moreover, the visual perception training providing apparatus 100 according to an embodiment of the present disclosure may expand the trainee's visual perception range by resetting the training point depending on the training result.

In the meantime, according to an embodiment, the visual perception training providing apparatus 100 may be implemented to perform a training point setting operation and a visual perception training operation through an external device. For example, when the visual perception training providing apparatus 100 is implemented as a server, the visual perception training providing apparatus 100 may perform the training point setting operation and a visual perception training operation by communicating with an external device through the communication module 110. In this case, an operation of using the input module 130 and the display module 150 of the visual perception training providing apparatus 100 described in this specification may be replaced with an operation of using a display module and an input module of the external device through the communication module 110.

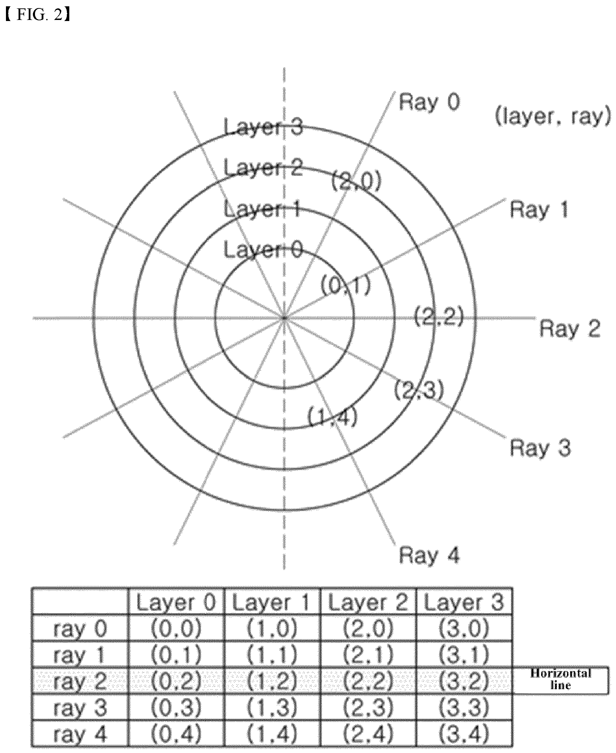

FIG. 2 is a diagram explaining a circular layer coordinate system, according to an embodiment of the present disclosure. Referring to FIG. 2, the circular layer coordinate system may include first to fourth circular layers Layer 0, Layer 1, Layer 2, and Layer3, which have different radius sizes based on the origin, and first to fifth straight line Ray 0, Ray 1, Ray 2, Ray 3, and Ray 4 passing through the origin.

In an embodiment, the first circular layer Layer 0 among the first to fourth circular layers Layer 0, Layer 1, Layer 2, and Layer3 is a circular layer with the smallest radius, and the radius increases as it goes to the fourth circular layer Layer 3. In an embodiment, the radius sizes of circular layers may be set such that an internal area from the origin to the fourth circular layer Layer 3 corresponds to the center vision within the trainee's visual range, and an outer area of the fourth circular layer Layer 3 corresponds to the peripheral vision within the trainee's visual range.

In an embodiment, each of the first to fifth straight line Ray 0, Ray 1, Ray 2, Ray 3, and Ray 4 may have an angle for dividing the half side of the circular layer coordinate system by 30 degrees. For example, referring to FIG. 2, with respect to the half side including the first and fourth quadrants of the circular layer coordinate system, the first to fifth straight line Ray 0, Ray 1, Ray 2, Ray 3, and Ray 4 have 30 degrees, 60 degrees, 90 degrees, 120 degrees, and 150 degrees from the longitudinal axis passing through the origin, respectively. In this specification, the third straight line Ray 2 may be referred to as a "horizontal line".

In an embodiment, the circular layer coordinate system may include a plurality of cross points where the first to fourth circular layer Layer 0, Layer 1, Layer 2, and Layer3 cross the first to fifth straight line Ray 0, Ray 1, Ray 2, Ray 3, and Ray 4. Moreover, coordinates of a plurality of cross points may be expressed as (a circular layer or a straight line).

For example, a cross point where the first circular layer Layer 0 intersects the second straight line Ray 1 may be expressed as (0,1). Besides, a cross point where the third circular layer Layer 2 intersects the first straight line Ray 0 may be expressed as (2,0). Moreover, a cross point where the third circular layer Layer 2 intersects the third straight line Ray 2 may be expressed as (2,2). Furthermore, a cross point where the third circular layer Layer 2 intersects the fourth straight line Ray 3 may be expressed as (2,3). In addition, a cross point where the second circular layer Layer 1 intersects the fifth straight line Ray 4 may be expressed as (1,4).

In an embodiment, the visual perception training providing apparatus 100 may set a training point by using only the half side of the circular layer coordinate system. In particular, the visual perception training providing apparatus 100 may set a training point by using only the half side corresponding to the trainee's visual impairment area. For example, when the trainee's visual impairment area is placed on the right side, the visual perception training providing apparatus 100 may only use the right half side of the circular layer coordinate system. Moreover, when the trainee's visual impairment area is placed on the left side, the visual perception training providing apparatus 100 may only use the left half side of the circular layer coordinate system. In the meantime, the present disclosure is not limited thereto. It is obvious that the visual perception training providing apparatus 100 may be implemented to set a training point by using the quadrant or all sides of the circular layer coordinate system.

In the meantime, FIG. 2 illustrates and describes that the circular layer coordinate system includes four circular layers and five straight lines, but the present disclosure is not limited thereto. It is obvious that the number of circular layers and the number of straight lines constituting the circular layer coordinate system may be smaller or greater than the examples.

Furthermore, FIG. 2 illustrates and describes that a plurality of straight lines are straight lines passing through the origin, but the present disclosure is not limited thereto. In an embodiment, the plurality of straight lines may be implemented as straight lines that do not pass through the origin and are horizontally or vertically parallel to each other.

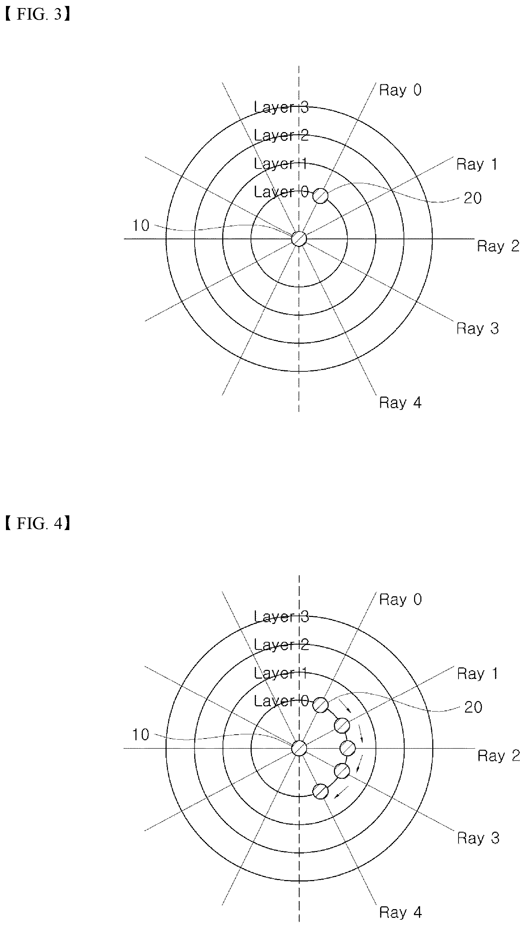

FIG. 3 is a diagram for describing a training point setting operation using a circular layer coordinate system, according to an embodiment of the present disclosure.

In an embodiment, the visual perception training providing apparatus 100 may perform a test for identifying a trainee's visual perception range to set a training point. The visual perception training providing apparatus 100 may perform a test on each of a plurality of cross points.

In particular, the testing of the visual perception training providing apparatus 100 may begin with displaying a circular layer coordinate system through the display module 150. Moreover, the visual perception training providing apparatus 100 may display the first reference image 10 on the origin and may display the second reference image 20 at a cross point on a circular layer. The first reference image 10 and the second reference image 20 may be the same as or different from each other. In an embodiment, the first reference image 10 and the second reference image 20 may be one of garbor patch images.

In a state where the trainee looks at the first reference image 10 located at the origin, the visual perception training providing apparatus 100 may ask the trainee to indicate whether the second reference image 20 located at a specific cross point is the same image as the first reference image 10. Furthermore, the visual perception training providing apparatus 100 may determine whether the trainee's response is correct. Also, the test of the visual perception training providing apparatus 100 is terminated.

For example, when the first reference image 10 and the second reference image 20 are the same as (or different from) each other and the trainee sends a response indicating that they are the same as (different from) each other, the visual perception training providing apparatus 100 may determine that the response is the correct answer. Besides, when the first reference image 10 and the second reference image 20 are the same as (or different from) each other, and the trainee sends a response indicating that they are different from (the same as) each other, the visual perception training providing apparatus 100 may determine that the response is a wrong answer.

The visual perception training providing apparatus 100 may perform a test on each cross point multiple times and may calculate a response score for the corresponding cross point based on a response to the test performed multiple times. For example, the visual perception training providing apparatus 100 may perform a test on one cross point 12 times and may calculate the number of correct answers in 12 responses as a response score for the corresponding cross point. For example, when the number of correct answers in 12 responses is 12, the response score is 12 points. When the number of incorrect answers in 12 responses is 12, the response score is 0 points.

When the response score of the cross points falls within a valid score range, the visual perception training providing apparatus 100 may determine that a cross point falling within the valid score range is a candidate for a training point. Here, the valid score range refers to a score range determined to be capable of visual perception at a certain level through the tests performed multiple times, and may be set to a score range around 75% of the number of tests according to an embodiment. For example, when 12 tests are performed, a point between 8 and 10 may be set to a valid score range.

In other words, when a response score exceeds the valid score range, the trainee's visual perception is sufficiently possible, and thus the visual perception training providing apparatus 100 determines that that visual training is unnecessary. Moreover, when the response score does not exceed the valid score range, the trainee's visual perception may be impossible, and thus the visual perception training providing apparatus 100 may determine that the priority of the need for visual training is low.

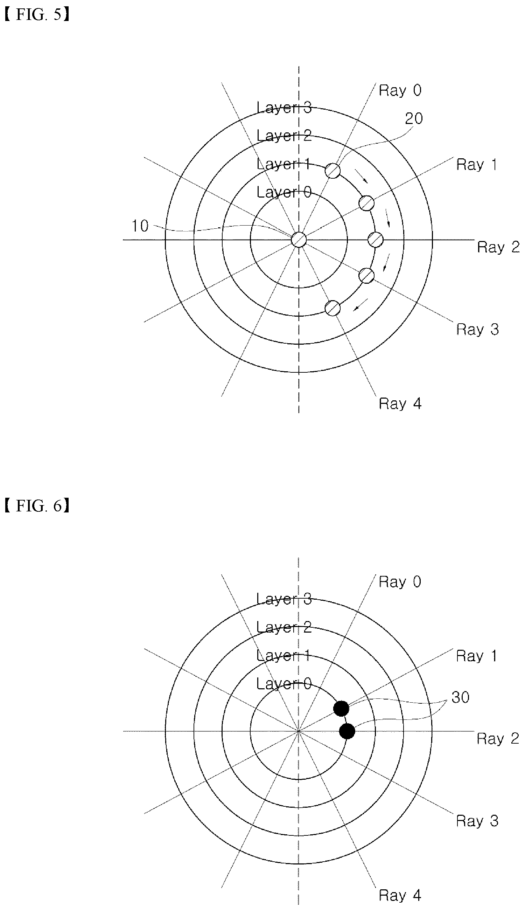

The visual perception training providing apparatus 100 may determine cross points, which fall within the valid score range, from among response scores of a plurality of cross points as a candidate for a training point. Moreover, when the number of candidates is the predetermined number, the visual perception training providing apparatus 100 may set the corresponding candidates as training points. For example, when there are two candidates, the visual perception training providing apparatus 100 may set the two candidates as training points. When 0, 1, or 2 or more candidates are present, detailed descriptions of a method in which the visual perception training providing apparatus 100 selects training points will be given later with reference to FIGS. 6 to 10.

FIG. 4 is a diagram illustrating a training point setting operation in a first circular layer, according to an embodiment of the present disclosure.

In an embodiment, the visual perception training providing apparatus 100 may search for a training point starting from a circular layer closest to the origin. Referring to FIG. 4, the visual perception training providing apparatus 100 may search for a training point starting from the first circular layer Layer 0, which is closest to the origin.

In particular, the visual perception training providing apparatus 100 may perform a test on cross points on the first circular layer Layer 0 multiple times. For example, referring to FIG. 4, the visual perception training providing apparatus 100 may sequentially test each of the five cross points (0,0), (0,1), (0,2), (0,3), and (0,4) on the first circular layer Layer 0 multiple times.

Moreover, the visual perception training providing apparatus 100 may calculate a response score of each of five cross points (0,0), (0,1), (0,2), (0,3), and (0,4) on the first circular layer Layer 0 based on a trainee's response.

When the number of cross points, which fall within the valid score range, from among the five cross points (0,0), (0,1), (0,2), (0,3), and (0,4) corresponds to the predetermined number (e.g., 2), the visual perception training providing apparatus 100 may set the cross points as training points. However, when the number of cross points, which belong to the valid score range, from among the five cross points (0,0), (0,1), (0,2), (0,3), and (0,4) is smaller than the predetermined number (e.g., 0 or 1), the visual perception training providing apparatus 100 may search for the next circular layer.

FIG. 5 is a diagram illustrating a training point setting operation in a second circular layer, according to an embodiment of the present disclosure.

Referring to FIG. 5, the visual perception training providing apparatus 100 may search for a training point on the second circular layer Layer 1, which is a circular layer second closest to the origin, compared with the first circular layer Layer 0 closest to the origin.

In particular, the visual perception training providing apparatus 100 may perform a test on cross points on the second circular layer Layer 1 multiple times. For example, referring to FIG. 5, the visual perception training providing apparatus 100 may sequentially test each of the five cross points (1,0), (1,1), (1,2), (1,3), and (1,4) on the second circular layer Layer 1 multiple times.

Moreover, the visual perception training providing apparatus 100 may calculate a response score of each of five cross points (1,0), (1,1), (1,2), (1,3), and (1,4) on the second circular layer Layer 1 based on a trainee's response.

When the number of cross points, which fall within the valid score range, from among ten cross points (0,0), (0,1), (0,2), (0,3), (0,4), (1,0), (1,1), (1,2), (1,3), and (1,4) on the first circular layer Layer 0 and the second circular layer Layer 1 corresponds to the predetermined number (e.g., 2), the visual perception training providing apparatus 100 may set the cross points as training points. However, when the number of cross points, which belong to the valid score range, from among the ten cross points (0,0), (0,1), (0,2), (0,3), (0,4), (1,0), (1,1), (1,2), (1,3), and (1,4) is smaller than the predetermined number (e.g., 0 or 1), the visual perception training providing apparatus 100 may search for the next circular layer. This same method may be equally applied to the third circular layer Layer 2 and the fourth circular layer Layer 3.

FIG. 6 is a diagram illustrating a training point setting operation when two or more candidates are found, according to an embodiment of the present disclosure.

**In** an embodiment, the visual perception training providing apparatus 100 may discover the predetermined number (e.g., two) of candidates on one circular layer. For example, referring to FIG. 6, the visual perception training providing apparatus 100 may identify two cross points (0,1) and (0,2) having response scores falling within the valid score range on the first circular layer Layer 0. In this case, the visual perception training providing apparatus 100 may set the two cross points (0,1) and (0,2) as training points 30.

In an embodiment, the visual perception training providing apparatus 100 may discover more than two candidates. For example, the visual perception training providing apparatus 100 may discover three or more candidates on one circular layer. In this case, the visual perception training providing apparatus 100 may select the predetermined number (e.g., two) of three or more candidates based on a priority and may set the candidates as training points. Here, the priority may be lowered in the order of 'intermediate value-lower value-upper value' of a valid score range. For example, when the valid score range is from 8 points to 10 points, the priority may be lowered in the order of '9 points-8 points-10 points'. Accordingly, when three candidates respectively have response scores of 8 points, 9 points, and 10 points, the candidates with 9 points and 8 points may be set as training points.

In the meantime, the example of priority is not limited to the example. The priority may be set to be lowered in the order of 'intermediate value-top value-intermediate value' of the valid score range, or may be set according to other criteria.

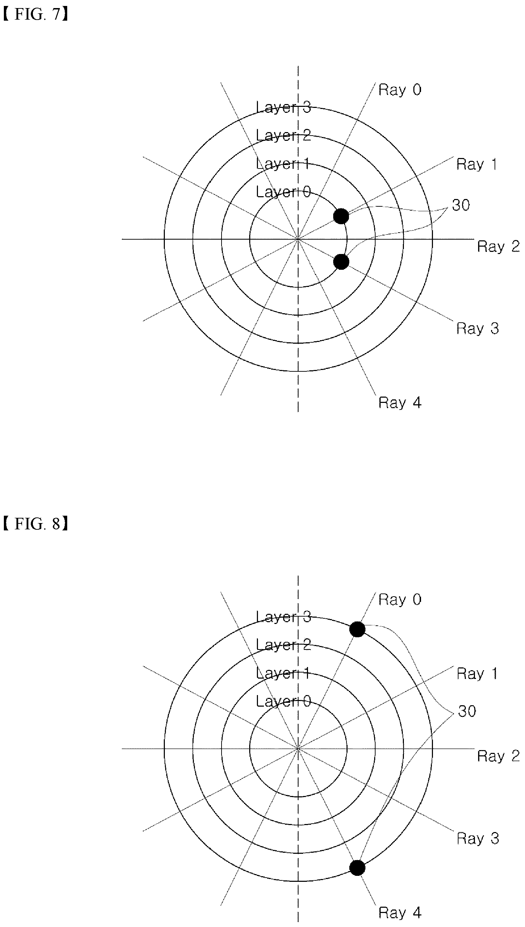

FIG. 7 is a diagram for describing a training point setting operation when one candidate is found, according to an embodiment of the present disclosure.

In an embodiment, the visual perception training providing apparatus 100 may search for all circular layers but found only one candidate. In this case, the visual perception training providing apparatus 100 may have different methods of setting training points based on whether one candidate is present on a horizontal line (i.e., the third straight line Ray 2).

In detail, when one candidate is not present on the horizontal line, the visual perception training providing apparatus 100 may determine a point, which is symmetrical to the candidate with respect to the horizontal line, as the second candidate. For example, referring to FIG. 7, when one candidate is (0,1), the visual perception training providing apparatus 100 may determine (0,3), which is symmetrical to (0,1) with respect to the horizontal line, as the second candidate. Moreover, the visual perception training providing apparatus 100 may set the two candidates (0,1) and (0,3) as training points.

Furthermore, when one candidate is present on the horizontal line, the visual perception training providing apparatus 100 may determine two points, which are symmetrical to each other with respect to the horizontal line, as candidates. In an embodiment, the visual perception training providing apparatus 100 may determine two points, which are symmetrical to each other with respect to the horizontal line, from among cross points present on a circular layer, on which one candidate is present, as candidates. For example, when one candidate is (0,2), the visual perception training providing apparatus 100 may set the two points (0,1) and (0,3), which are symmetrical to each other with respect to the horizontal line, from among cross points on the first circular layer Layer 0 as training points.

FIGS. 8 to 10 are diagrams for describing a training point setting operation when there is no candidate, according to an embodiment of the present disclosure.

**In** an embodiment, the visual perception training providing apparatus 100 may search for all circular layers but may not find a candidate falling within a valid score range. **In** this case, the visual perception training providing apparatus 100 may have different methods of setting training points depending on response scores of all cross points.

**In** detail, when the response scores of all the cross points exceed the valid score range, the visual perception training providing apparatus 100 may determine two points, which are furthest from each other, among cross points on the outermost circular layer as candidates. For example, referring to FIG. 8, the visual perception training providing apparatus 100 may determine two points (3,0) and (3,4), which are furthest from each other, from among cross points on the outermost fourth circular layer Layer 3 as candidates. Moreover, the visual perception training providing apparatus 100 may set the two candidates (3,0) and (3,4) as the training point 30.

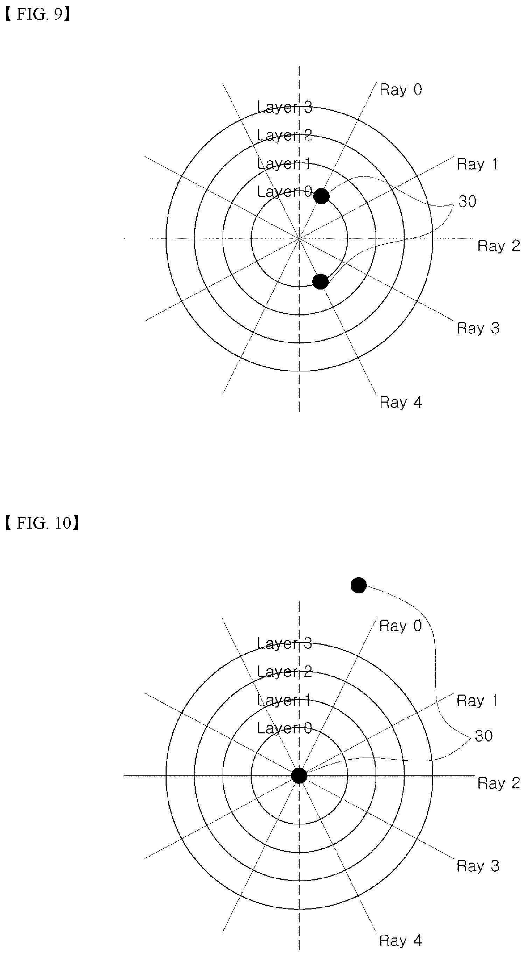

Furthermore, when the response scores of all the cross points do not exceed the valid score range, the visual perception training providing apparatus 100 may determine the two points, which are furthest from each other, from among cross points on the innermost circular layer as candidates. For example, referring to FIG. 9, the visual perception training providing apparatus 100 may determine two points (0,0) and (0,4), which are furthest from each other, from among cross points on the innermost first circular layer Layer 0 as candidates. Moreover, the visual perception training providing apparatus 100 may set the two candidates (0,0) and (0,4) as the training point 30.

In another embodiment, when the visual perception training providing apparatus 100 searches for all circular layers but does not find a candidate falling within the valid score range, the visual perception training providing apparatus 100 may set the origin and a point, which is present in the outer area of the outermost circular layer, to the training points 30. In an embodiment, the point in the outer area may be selected randomly or may be a predetermined point.

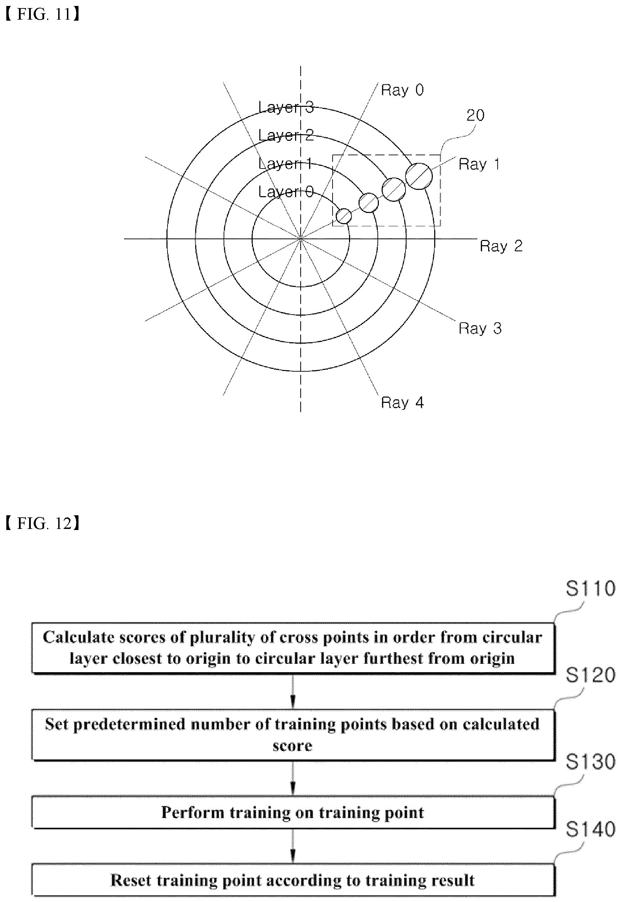

FIG. 11 is a diagram showing a size of a reference image for each circular layer, according to an embodiment of the present disclosure.

In an embodiment, as a circular layer where a cross point at which the second reference image 20 is displayed moves away from the origin, the size of the second reference image 20 may increase. For example, referring to FIG. 11, the second reference image 20 displayed at each of cross points on the first to fourth circular layers Layer 0, Layer 1, Layer 2, and Layer 3 is smallest on the first circular layer Layer 0. As it goes to the fourth circular layer Layer 3, the second reference image 20 becomes larger. The reason is that an average distance between cross points in a circular layer coordinate system increases as a distance from the origin increases.

In an embodiment, with respect to each of the first to fourth circular layers Layer 0, Layer 1, Layer 2, and Layer 3, information about a distance from the origin to a reference image and a size of a peripheral reference image is shown in Table 1 below.

**[Table 1]**

| Layer | | 0 | 1 | 2 | 3 |
|---|---|---|---|---|---|
| Distance (deg) from origin to reference image | deg | ~5.6 | ~8.5 | ~11.3 | ~14.1 |
| | Length (40m away) | ~4 | ~6 | ~8 | ~10 |
| Size (deg) of peripheral reference image | deg | 4 | 6 | 8 | 10 |
| | Length (40m away) | ~2.8 | ~42 | ~5.6 | ~7 |

FIG. 12 is a flowchart showing a visual perception training providing method, according to an embodiment of the present disclosure. Referring to FIG. 12, the visual perception training providing apparatus 100 may calculate a trainee's response score for each cross point in order from cross points on a circular layer closest to the origin to cross points on a circular layer furthest from the origin (S110). In detail, in a circular layer coordinate system where a plurality of circular layers intersects a plurality of straight lines, the visual perception training providing apparatus 100 may perform a test on each cross point in order from cross points on the circular layer closest to the origin to cross points on the circular layer furthest from the origin.

Here, the test may be an operation of receiving a response as to whether a first reference image displayed at the origin is the same as a second reference image displayed at a cross point. Accordingly, the score may be assigned based on whether a trainee's response is correct. The test may be performed multiple times. The visual perception training providing apparatus 100 may calculate the trainee's response score based on scores from multiple tests. Moreover, the visual perception training providing apparatus 100 may set the predetermined number of training points based on the calculated response score (S120). In detail, the visual perception training providing apparatus 100 identifies the number of scores, which fall within a valid score range, from among response scores for cross points on one circular layer. When the number of cross points belonging to the valid score range is the predetermined number (e.g., 2), the visual perception training providing apparatus 100 may set the corresponding cross points as training points. In the meantime, when the number of cross points belonging to the valid score range is smaller than the predetermined number, the visual perception training providing apparatus 100 identifies the number of scores, which fall within the valid score range, from among response scores including cross points on the next circular layer.

When the number of scores, which fall within the valid score range, from among response scores including cross points on the last circular layer is still smaller than the predetermined number, the visual perception training providing apparatus 100 may set a point, which is symmetrical to a cross point falling within the valid score range with respect to the horizontal line, as the training point. Moreover, when there is no score, which falls within the valid score range, from among the response scores including cross points on the last circular layer, the visual perception training providing apparatus 100 may set two points, which are furthest from each other on the innermost circular layer or the farthest circular layer, as the training points.

When the number of scores falling within the valid score range among the response scores for cross points on one circular layer is greater than the predetermined number, the visual perception training providing apparatus 100 may select the predetermined number of cross points according to a priority and may set the selected cross points as the training points. Here, the priority may be lowered in the order of 'intermediate value-lower value-upper value' of a valid score range.

Furthermore, the visual perception training providing apparatus 100 may perform visual perception training on a training point (S130). In detail, the visual perception training providing apparatus 100 may perform visual perception training of the trainee on the predetermined number of training points and may obtain the trainee's training result.

Also, the visual perception training providing apparatus 100 may reset the training point according to the training result (S140). In particular, when the training result for the existing training point is excellent, the visual perception training providing apparatus 100 may set a new training point to further expand the trainee's visual perception range. In the meantime, when the training result for the existing training point is insufficient, the visual perception training providing apparatus 100 may set another training point capable of expanding the visual perception range.

Meanwhile, the disclosed embodiments may be implemented in a form of a recording medium storing instructions executable by a computer. The instructions may be stored in a form of program codes, and, when executed by a processor, generate a program module to perform operations of the disclosed embodiments. The recording medium may be implemented as a computer-readable recording medium.

The computer-readable recording medium may include all kinds of recording media in which instructions capable of being decoded by a computer are stored. For example, there may be read only memory (ROM), random access memory (RAM), magnetic tape, magnetic disk, flash memory, optical data storage device, and the like.

Disclosed embodiments are described above with reference to the accompanying drawings. One ordinary skilled in the art to which the present disclosure belongs will understand that the present disclosure may be practiced in forms other than the disclosed embodiments without altering the technical ideas or essential features of the present disclosure. The disclosed embodiments are examples and should not be construed as limited thereto.

## Claims

1. A visual perception training providing apparatus, the apparatus comprising:
a display module configured to display a circular layer coordinate system, which includes a plurality of circular layers and a plurality of straight lines, and which includes a plurality of cross points where the plurality of circular layers intersect the plurality of straight lines;
a memory configured to store information about the circular layer coordinate system;
an input module configured to receive a response of a trainee; and
a control module configured to:
calculate response scores of the trainee for the plurality of cross points in order from a circular layer closest to an origin of the circular layer coordinate system to a circular layer furthest away from the origin;
set a predetermined number of a training point based on the calculated response scores; and
perform visual perception training on the training point.

2. The apparatus of claim 1, wherein the control module is configured to:
display a first reference image at the origin;
display a second reference image at each of the plurality of cross points;
receive a response of the trainee as to whether the first reference image is the same as the second reference image;
perform a test operation of determining whether the response of the trainee is correct; and
calculate the response scores for the plurality of cross points based on whether the response of the trainee is correct.

3. The apparatus of claim 2, wherein the control module is configured to:
perform the test operation multiple times on each of the plurality of cross points; and
calculate the response scores for the plurality of cross points based on a plurality of responses of the trainee for the test operation performed multiple times.

4. The apparatus of claim 3, wherein the control module is configured to:
select cross points, which have response scores falling within a valid score range, from among the plurality of cross points as candidates.

5. The apparatus of claim 4, wherein the control module is configured to:
select the candidates among cross points on a first circular layer among the plurality of circular layers;
when a number of candidates is smaller than the predetermined number, additionally select the candidates from cross points on a second circular layer, which is farther than the first circular layer, from among the plurality of circular layers; and
when the number of candidates is greater than the predetermined number, select the predetermined number of the candidates among the candidates depending on priorities and set the selected candidates as the training point.

6. The apparatus of claim 4, wherein the control module is configured to:
select the candidates among cross points on a first circular layer among the plurality of circular layers;
when a number of the candidates is the predetermined number, set the candidates to the training point.

7. The apparatus of claim 1, wherein the control module is configured to:
reset the training point based on the result of the visual perception training, and
wherein the plurality of straight lines pass through the origin of the circular layer coordinate system.

8. A visual perception training providing method performed by a control module of an apparatus, the method comprising:
displaying a circular layer coordinate system, which includes a plurality of circular layers and a plurality of straight lines, and which includes a plurality of cross points where the plurality of circular layers intersect the plurality of straight lines, on a display module of the apparatus;
calculating response scores of a trainee for the plurality of cross points in order from a circular layer closest to an origin of the circular layer coordinate system to a circular layer furthest away from the origin;
setting a predetermined number of a training point based on the calculated response scores; and
performing visual perception training on the training point.

9. The method of claim 8, wherein the control module is configured to:
display a first reference image at the origin;
display a second reference image at each of the plurality of cross points;
receive a response of the trainee as to whether the first reference image is the same as the second reference image;
perform a test operation of determining whether the response of the trainee is correct; and
calculate the response scores for the plurality of cross points based on whether the response of the trainee is correct.

10. The method of claim 9, wherein the control module is configured to:
perform the test operation multiple times on each of the plurality of cross points; and
calculate the response scores for the plurality of cross points based on a plurality of responses of the trainee for the test operation performed multiple times.

11. The method of claim 10, wherein the control module is configured to:
select cross points, which have response scores falling within a valid score range, from among the plurality of cross points as candidates.

12. The method of claim 11, wherein the control module is configured to:
select the candidates among cross points on a first circular layer among the plurality of circular layers;
when a number of candidates is smaller than the predetermined number, additionally select the candidates from cross points on a second circular layer, which is farther than the first circular layer, from among the plurality of circular layers; and
when the number of candidates is greater than the predetermined number, select the predetermined number of the candidates among the candidates depending on priorities and set the selected candidates as the training point.

13. The method of claim 11, wherein the control module is configured to:
select the candidates among cross points on a first circular layer among the plurality of circular layers;
when a number of the candidates is the predetermined number, set the candidates to the training point.

14. The method of claim 8, wherein the control module is configured to:
reset the training point based on the result of the visual perception training, and
wherein the plurality of straight lines pass through the origin of the circular layer coordinate system.

15. A computer-readable recording medium storing a computer program for performing the visual perception training providing method of claim 8.
